# EUROPEAN PATENT SPECIFICATION

(11) **EP 2 389 160 B1**
(45) Date of publication and mention of the grant of the patent: **13.09.2017**
(21) Application number: 10701599.2
(22) Date of filing: 22.01.2010
(51) Int. Cl.: A61K 9/50, A61K 9/16

(54) **CONTINUOUS DOUBLE EMULSION PROCESS FOR MAKING MICROPARTICLES**
KONTINUIERLICHES DOPPELEMULSIONSVERFAHREN ZUR HERSTELLUNG VON MIKROTEILCHEN
PROCÉDÉ CONTINU PAR ÉMULSION DOUBLE DE FABRICATION DE PARTICULES

(30) Priority: 23.01.2009 US 146884 P
(43) Date of publication of application: 30.11.2011
(73) Proprietor: Evonik Corporation, Parsippany NJ 07054 (US)
(72) Inventor: MARKLAND, Peter, Birmingham, AL 35244 (US)
(74) Representative: Leißler-Gerstl, Gabriele
(86) International application number: PCT/US2010/021742
(87) International publication number: WO 2010/085607

(56) References cited:
- WO-A1-2007/058462
- WO-A2-00/66087
- DE-A1- 10 045 374
- FREITAS S ET AL: "Flow-through ultrasonic emulsification combined with static micromixing for aseptic production of microspheres by solvent extraction" EUROPEAN JOURNAL OF PHARMACEUTICS AND BIOPHARMACEUTICS, ELSEVIER SCIENCE PUBLISHERS B.V., AMSTERDAM, NL LNKD- DOI:10.1016/J.EJPB.2005.05.004, vol. 61, no. 3, 1 October 2005 (2005-10-01), pages 181-187, XP025317686 ISSN: 0939-6411 [retrieved on 2005-10-01]
- KATOU H ET AL: "Kinetics of solvent extraction/evaporation process for PLGA microparticle fabrication" 19 November 2008 (2008-11-19), INTERNATIONAL JOURNAL OF PHARMACEUTICS, ELSEVIER BV, NL LNKD- DOI:10.1016/J.IJPHARM.2008.08.015, PAGE(S) 45 - 53 , XP025562418 ISSN: 0378-5173 [retrieved on 2008-08-22] * abstract
- FREITAS S ET AL: "Microencapsulation by solvent extraction/evaporation: reviewing the state of the art of microsphere preparation process technology" JOURNAL OF CONTROLLED RELEASE, ELSEVIER, AMSTERDAM, NL LNKD- DOI:10.1016/J.JCONREL.2004.10.015, vol. 102, no. 2, 2 February 2005 (2005-02-02), pages 313-332, XP004710961 ISSN: 0168-3659

## Description

### BACKGROUND

Preparation of microparticles containing water-soluble bioactive agents therein is an attractive alternative to conventional dosage forms. Microparticle encapsulation offers numerous advantages, such as reduced toxicity, and improved efficacy, among others. Many microparticle encapsulation protocols also provide an improved pharmacological drug release profile and as such can provide for improved patient compliance.

Various methods exist for encapsulating a water-soluble bioaetive agent within a microparticle. A few examples are the double emulsion liquid extraction process, organic phase seperation methods, supercritical fluid methods, and spray drying methods. Oftentimes, however, traditional methods rely on discontinous processing, wherein single phases, solutions, or dispersions are prepared, mixed, and/or processed separately from other phases, * solutions, or dispersions. Thus, such processes can be bound by cumbersome time constraints, as well as other challenges that typically arise during discontinous processing.

As such, a need exists for improved processes that provide a more practical and economical approach to microparticle encapsulation. These needs and other needs are satisfied by the present invention.

Freitas et al., in "Flow-through ultrasonic emulsification combined with static micromixing for aseptic production of microspheres by solvent extraction", Eur J Pharm Biopharm. 2005 Oct;61(3):181-7, discloses a process that utilizes flow-through ultrasonic emulsification for the preparation of the primary emulsion (W₁/O) in combination with a static micromixer for the production of the double emulsion (W₁/O/W₂). The disclosed process for making microparticles occurs in three distinct steps.

Katou et al., in "Kinetics of solvent extraction/evaporation process for PLGA microparticle fabrication", Int J Pharm. 2008 Nov 19;364(1):45-53, discloses the kinetics of solvent extraction and evaporation from an o/w dispersion. Specifically, a mathematical model describing the kinetics of solvent transport from the o-phase into the surrounding w-phase is disclosed. Furthermore, a five-step process for forming microparticles is disclosed.

Freitas et al., in "Microencapsulation by solvent extraction/evaporation: reviewing the state of the art of microsphere preparation process technology", J Control Release. 2005 Feb 2;102(2):313-32, discloses microencapsulation by solvent extraction/evaporation and generally discusses a water-in-oil-in-water double emulsion approach to microencapsulation.

DE 100 45 374 A1 discloses a process for preparing microparticles that utilizes two primary oil phases or emulsions. To overcome perceived disadvantages of the W₁/O/W₂ double emulsification and the O/W emulsification techniques for microparticle formation, encapsulation of the same active ingredient in at least two individual polymers is disclosed.

WO 00/66087 discloses an emulsion-based process for making microparticles. The disclosed process utilizes a dispersed phase, a continuous phase, and an extraction phase. The steps for the formation of the emulsion are disclosed as follows: first, the dispersed phase is formed; second, the active agent is added to the dispersed phase; third, the continuous phase is formed; and fourth, the dispersed phase is mixed with the continuous phase, which mixing can be done continuously or batchwise.

WO 2007/058642 A1 discloses a method of preparing microparticles comprising the formation of a double emulsion. The process involves the batch-wise preparation of the various phases. A step-by-step or serial process for microparticle formation is disclosed.

### SUMMARY

Described herein are methods for forming microparticles comprising: (a) providing a first phase comprising a bioactive agent dissolved or dispersed in a first aqueous phase; (b) providing a second phase comprising a polymer dissolved or dispersed in an organic phase; (c)^{#}mixing the first and second phases to form a water-in-oil emulsion; (d)^{#}mixing the emulsion of step (c) with a second aqueous phase to form a water-in-oil-in-water double emulsion; and (e) removing the organic to form the microparticles. Also disclosed are microparticles made by the disclosed methods. #: continuously

The advantages of the invention will be set forth in part in the description which follows, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### BRIEF DESCRIPTION OF THE DRAWINGS

**Figures 1a****-c** show SEM and confocal raman images of exemplary microparticle cross-sections of microparticles made by the discontinuous process described in Example 1.
**Figures 2a****-c** show SEM images of cross sections of microparticles collected from (a) early, (b) middle, and (c) late portion of the run from Example 1 (discontinuous double-emulsion process using 12,000 RPM mixing energy to prepare the primary emulsion) (250x magnification).
**Figures 3a****-c** show SEM images of cross sections of microparticles collected from (a) early, (b) middle, and (c) late portion of the run from Example 2 (continuous double-emulsion process using 12,000 RPM mixing energy to prepare the primary emulsion) (250x magnification).
**Figures 4a****-c** show SEM images of cross sections of microparticles collected from (a) early, (b) middle, and (c) late portion of the run from Example 3 (discontinuous double-emulsion process using a high ratio of inner-phase water to prepare the primary emulsion) (250x magnification).
**Figures 5a****-c** show SEM images of cross sections of microparticles collected from (a) early, (b) middle, and (c) late portion of the run from Example 4 (continuous double-emulsion process using a high ratio of inner-phase water to prepare the primary emulsion) (250x magnification).

### DETAILED DESCRIPTION

Before the present compounds, compositions, composites, articles, devices, methods, or uses are disclosed and described, it is to be understood that the aspects described below are not limited to specific compounds, compositions, composites, articles, devices, methods, or uses as such may, of course, vary. It is also to be understood that the terminology used herein is for the purpose of describing particular aspects only and is not intended to be limiting.

In this specification and in the claims that follow, reference will be made to a number of terms that shall be defined to have the following meanings:
Throughout this specification, unless the context requires otherwise, the word "comprise," or variations such as "comprises" or "comprising," will be understood to imply the inclusion of a stated integer or step or group of integers or steps but not the exclusion of any other integer or step or group of integers or steps.

It must be noted that, as used in the specification and the appended claims, the singular forms "a," "an" and "the" include plural referents unless the context clearly dictates otherwise. Thus, for example, reference to "a bioactive agent" includes mixtures of two or more such agents, and the like.

"Optional" or "optionally" means that the subsequently described event or circumstance can or cannot occur, and that the description includes instances where the event or circumstance occurs and instances where it does not.

Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

A weight percent of a component, unless specifically stated to the contrary, is based on the total weight of the formulation or composition in which the component is included.

The term "microparticle" is used herein to refer generally to a variety of structures having sizes from about 10 nm to 2000 microns (2 millimeters) and includes microcapsule, microsphere, nanoparticle, nanocapsule, nanosphere as well as particles, in general, that are less than about 2000 microns (2 millimeters). In one aspect, the bioactive agent is encapsulated in the microparticle.

The term "biocompatible" refers a substance that is substantially non-toxic to a subj ect.

"Biodegradable" is generally referred to herein as a material that will erode to soluble species or that will degrade under physiologic conditions to smaller units or chemical species that are, themselves, non-toxic (biocompatible) to the subject and capable of being metabolized, eliminated, or excreted by the subject.

A "bioactive agent" refers to an agent that has biological activity. The biological agent can be used to treat, diagnose, cure, mitigate, prevent (*i.e.,* prophylactically), ameliorate, modulate, or have an otherwise favorable effect on a disease, disorder, infection, and the like. A "releasable bioactive agent" is one that can be released from a disclosed microparticle. Bioactive agents also include those substances which affect the structure or function of a subject, or a pro-drug, which becomes bioactive or more bioactive after it has been placed in a predetermined physiological environment.

Disclosed are compounds, compositions, and components that can be used for, can be used in conjunction with, can be used in preparation for, or are products of the disclosed methods and compositions. These and other materials are disclosed herein, and it is understood that when combinations, subsets, interactions, groups, *etc.* of these materials are disclosed that while specific reference of each various individual and collective combinations and permutation of these compounds may not be explicitly disclosed, each is specifically contemplated and described herein. For example, if a number of different polymers and agents are disclosed and discussed, each and every combination and permutation of the polymer and agent are specifically contemplated unless specifically indicated to the contrary. Thus, if a class of molecules A, B, and C are disclosed as well as a class of molecules D, E, and F and an example of a combination molecule, A-D is disclosed, then even if each is not individually recited, each is individually and collectively contemplated. Thus, in this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E; B-F, and C-E are specifically contemplated and should be considered disclosed from disclosure of A, B, and C; D, E, and F; and the example combination A-D. This concept applies to all aspects of this disclosure including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

In one aspect, the disclosed methods comprise continous steps for microparticle encapsulation, wherein each mixing step or feeding step is continous with the next. Thus, in one aspect, the present methods avoid discontinous (e.g., batch) processing, wherein single phases, solutions, or dispersions are prepared, mixed, and/or processed separately from other phases, solutions, or dispersions, and subsequently mixed together. It will be apparent that the use of a continous process enables a more cost efficient and timely approach to microparticle encapsulation. Additionally, less bioactive agent is lost due to the quicker production times afforded by the disclosed methods, thereby leading to higher bioactive agent loading efficiency. Further, control and reproducibility of the primary emulsion (water-in-oil-emulsion) can be achieved using a continuous process, which provides potential for improving the control and reproducibility of the final microparticle product. Surprisingly, the microparticles of the present invention, made by the continuous process of the invention herein, exhibit reduced initial burst. As will be apparent, the improved and relatively consistent morphology of the particles formed by the continuous double-emulsion process described herein resulted in a reduction in initial burst for a bioactive agent encapsulated in the microparticle. This was likely a result of the controlled and improved distribution of the bioactive agent throughout the polymer matrix, in addition to excellent reproducibility and consistency in bioactive agent distribution and particle attributes, which were well-maintained throughout the continuous process. It will also be apparent that microparticles formed from the continuous double-emulsion process described herein are more consistent in their cross-sectional appearance and morphology than microparticles formed from a discontinuous (*e*.*g*., batch) process.

In one aspect, the method as defined in the claims for forming microparticles comprises: (a) providing a first phase comprising a bioactive agent dissolved or dispersed in a first aqueous phase; (b) providing a second phase comprising a polymer dissolved or dispersed in an organic phase; (c)^{#}mixing the first and second phases to form a water-in-oil emulsion; (d)^{#}mixing the emulsion of step (c) with a second aqueous phase to form a water-in-oil-in-water double emulsion; and (e) removing the organic to form the microparticles. #: continuously

The first phase comprising the first aqueous phase having the bioactive agent dissolved or dispersed therein can be provided using any suitable means. In one aspect, a desired quantity of the bioactive agent is dissolved or dispersed in a suitable aqueous solution or solvent. Any quantity of the bioactive agent can be used, depending on the desired loading of the bioactive agent in the microparticle. Likewise, any suitable volume of the aqueous solution or solvent can be used. The bioactive agent can be present in the aqueous phase in any desired weight %. For example, the bioactive agent can be present in the first aqueous phase in about 1% to about 90% by weight, including without limitation, about 5%, 10%, 15%, 20%, 30%, 40%, 50%, 60%, 70%, or 80% by weight.

The first phase can comprise any suitable aqueous solvent. Preferably, the aqueous solvent is one that will not substantially alter the composition of the bioactive agent. One non-limiting example of an aqueous solvent is water. In one aspect, water can be mixed with another miscible solvent, for example, ethanol, methanol, DMSO, DMF, isopropyl alcohol, among many other water-miscible polar solvents. In various aspects, the first phase can contain other excipients, such as buffers, salts, sugars, surfactants and/or viscosity-modifying agents, or combinations thereof. In a specific aspect, the first phase can comprise water and ethyl acetate.

The second phase can be provided by mixing the polymer in a suitable organic solvent. Generally, the organic solvent can be selected based on the polymer solubility or polymer dispersability in that solvent. Additionally, the organic solvent can be selected based on its immiscibility with the aqueous phase. Thus, a wide variety of organic solvents can be used. Non-limiting examples include ethyl acetate, chlorinated solvents such as methylene chloride, hexanes, or a combination thereof, among many other water immiscible organic solvents. The polymer can be present in the second phase in any desired weight %. For example, the polymer can be present in the second phase in about 1% to about 90% by weight, including without limitation, about 5%, 10%, 15%, 20%, 30%, 40%, 50%, 60%, 70%, or 80% by weight. The second phase can further comprise additives such as cosolvents, surfactants, emulsifiers, blends of two or more polymers, or a combination thereof, among other additives.

The first and second phases are continuously mixed to form a water-in-oil emulsion. The water-in-oil emulsion comprises the first aqueous phase comprising the bioactive agent as the internal phase, which is substantially surrounded by the oil phase, comprising the second phase containing the polymer. In one aspect, the formation of the water-in-oil emulsion can be aided by a mixer, which is typically in-line with the continuous process. In one aspect, for example, the first phase and second phase can independently flow through feed lines that lead to a continous mixer. The continous mixer can comprise any suitable mixing means, including a static and/or dynamic mixer. The mixer can be any mixer comprising mechanical or non-mechanical mixing parts. In one aspect, the continous mixer comprises a static mixer having static mixing arms that create turbulance in the flow such that the first and second phase are mixed to thereby form the water-in-oil emulsion. In other aspects, the continuous mixer comprises an emulsifier, an emulsification device, or a homogenizer (*e.g.,* an in-line or continuous homogenizer or a rotor/stator homogenizer). Examples include without limitation packed-bed emulsifiers, screen, and membrane emulsifiers. In one aspect, the continuous mixer has mixing parts that can mix the phases at a desired revolutions per minute, such as from about 8,000 rpm to about 15,000 rpm, including for example, 12,000 rpm.

The water-in-oil emulsion (*i.e.,* the primary emulsion) is then continuously mixed with a second phase comprising a second aqueous phase to form a water-in-oil-in-water double emulsion. Once formed, the water-in-oil emulsion is typically immediately fed in a continous manner to the second continous process, thereby minimizing the loss of bioactive agent from the first aqueous phase. The water-in-oil emulsion comprises the first aqueous phase comprising the bioactive agent as the internal phase, which is substantially surrounded by the oil phase, comprising the second phase containing the polymer, the second phase being substantially surrounded by the second aqueous phase. The second aqueous phase is typically referred to as the continous-processing medium. Thus, for example, water, as the second aqueous phase, can be introduced into the water-in-oil emulsion by feeding water into the in-line process after the water-in-oil emulsion has passed through the first continous mixer.

The continuous processes can be done quickly, on the order of seconds or even less than one second per step. The formation of the water-in-oil-in-water double emulsion in the second continuous process can be aided by the use of a second static or dynamic continous mixer, including any of those mixers described above. The first and second mixers can be separate mixing devices and can comprise the same or different types of mixing devices. Or, the first and second mixing devices can be one continous mixing device having staged feed points along the traverse direction of the device for the various phases being mixed so that the first and second continuous processes are all performed in one continuous mixing device.

The second aqueous phase can comprise any desirable aqueous solvent. One non-limiting example of an aqueous solvent is water. In one aspect, water can be mixed with another miscible solvent, semi-miscible solvent, or low water-miscible solvent, for example, ethanol, methanol, DMSO, DMF, isopropyl alcohol, ethyl acetate, dichloromethane, etc, among many other water-miscible polar solvents, semi-miscible solvents, or low water-miscible solvents. In one aspect, the second aqueous phase can comprise a stabilizer that stabilizes the double emulsion. Non-limiting examples of suitable stabilizers include surfactants or emulsification aids such as poly(vinyl alcohol), PVA, or polysorbate surfactants or poloxamers. The first or second aqueous phase can also comprise an emulsifier, which aids in the formation of the emulsion or double-emulsion. A non-limiting example of an emulsifier is the surfactant Tween 80 or the poloxamer Pluronics F168. In further aspects, the second aqueous phase can comprise other additives such as an organic solvent, a cosolvent, a buffer, a salt, a sugar, or a combination thereof. In one particular aspect, the second aqueous phase comprises an organic solvent in addition to an added salt (such as sodium chloride).

Once the water-in-oil-in-water double emulsion is formed, microparticles can be formed from the double emulsion. The microparticles are typically formed by removing the organic solvent. The organic solvent can be removed by any suitable methods. In one aspect, the organic can be removed by extracting the organic with an extraction liquid, such as water. In other aspects, the organic can be removed by drying, such as by spray drying, drying under reduced pressure, solvent evaporation, or a combination thereof. Typically, the process of removing the organic should be done quickly to minimize the loss of bioactive agent from the first aqueous phase. The removal can also be performed using a continous process, such as a continous liquid extraction process.

The continous process of steps (a)-(d) can be applied to an oil-in-water-in-oil emulsion process. For example, a bioactive agent can be dissolved or dispersed in an oil phase, and a polymer can be dissolved or dispersed in a water phase. Next, an organic solvent can be added to form a double emulsion. Any of the above process steps described above can be nsed in accordance with the disclosed oil-in-water-in-oil double emulsion process.

A wide variety of polymers can be used with the methods disclosed herein. In one aspect, the desired release profile of the bioactive agent can influence the selection of the polymer. A biocompatible polymer, for example, can be selected so as to release or allow the release of a bioactive agent therefrom at a desired lapsed time after the microparticle has been administered to a subject. For example, the polymer can be selected to release or allow the release of the bioactive agent prior to the bioactive agent beginning to diminish its activity, as the bioactive agent begins to diminish in activity, when the bioactive agent is partially diminished in activity, for example at least 25%, at least 50% or at least 75% diminished, when the bioactive agent is substantially diminished in activity, or when the bioactive agent is completely gone or no longer has activity.

When a biodegradable polymer is used, the microparticle can be formulated so as to degrade within a desired time interval, once present in a subject. In some aspects, the time interval can be from about less than one day to about 1 month. Longer time intervals can extend to 6 months, including for example, polymer matrices that degrade from about ≥0 to about 6 months, or from about 1 to about 6 months. In other aspects, the polymer can degrade in longer time intervals, up to 2 years or longer, including, for example, from about ≥0 to about 2 years, or from about 1 month to about 2 years.

Non-limiting examples of suitable polymers include polyesters, polyhydroxyalkanoates, polyhydroxybutyrates, polydioxanones, polyhydroxyvalerates, polyanhydrides, polyorthoesters, polyphosphazenes, polyphosphates, polyphosphoesters, polydioxanones, polyphosphoesters, polyphosphates, polyphosphonates, polyphosphates, polyhydroxyalkanoates, polycarbonates, polyalkylcarbonates, polyorthocarbonates, polyesteramides, polyamides, polyamines, polypeptides, polyurethanes, polyalkylene alkylates, polyalkylene oxalates, polyalkylene succinates, polyhydroxy fatty acids, polyacetals, polycyanoacrylates, polyketals, polyetheresters, polyethers, polyalkylene glycols, polyalkylene oxides, polyethylene glycols, polyethylene oxides, polypeptides, polysaccharides, or polyvinyl pyrrolidones. Other non-biodegradable but durable polymers include without limitation ethylene-vinyl acetate co-polymer, polytetrafluoroethylene, polypropylene, polyethylene, and the like. Likewise, other suitable non-biodegradable polymers include without limitation silicones and polyurethanes.

In a further aspect, the polymer can be a polysaccharide, including modified or substituted forms of polysaccharides. Examples include without limitation maltodextrin, including both modified and substituted forms of a maltodextrin, starches, glycogen, cellulose, chitin, chitosan, dextrin, dextrans, glycans, glucans, hyalurorans, and modified or substituted versions thererof.

In a further aspect, the polymer can be a poly(lactide), a poly(glycolide), a poly(lactide-co-glycolide), a poly(caprolactone), a poly(orthoester), a poly(phosphazene), a poly(hydroxybutyrate) or a copolymer containing a poly(hydroxybutarate), a poly(lactide-co-caprolactone), a polycarbonate, a polyesteramide, a polyanhydride, a poly(dioxanone), a poly(alkylene alkylate), a copolymer of polyethylene glycol and a polyorthoester, a biodegradable polyurethane, a poly(amino acid), a polyamide, a polyesteramide, a polyetherester, a polyacetal, a polycyanoacrylate, a poly(oxyethylene)/poly(oxypropylene) copolymer, polyacetals, polyketals, polyphosphoesters, polyhydroxyvalerates or a copolymer containing a polyhydroxyvalerate, polyalkylene oxalates, polyalkylene succinates, poly(maleic acid), and copolymers, terpolymers, combinations, or blends thereof.

In a still further aspect, useful biocompatible polymers are those that comprise one or more residues of lactic acid, glycolic acid, lactide, glycolide, caprolactone, hydroxybutyrate, hydroxyvalerates, dioxanones, polyethylene glycol (PEG), polyethylene oxide, or a combination thereof. In a still further aspect, useful biocompatible polymers are those that comprise one or more residues of lactide, glycolide, caprolactone, or a combination thereof.

In one aspect, useful biodegradable polymers are those that comprise one or more blocks of hydrophilic or water soluble polymers, including, but not limited to, polyethylene glycol, (PEG), or polyvinyl pyrrolidone (PVP), in combination with one or more blocks another biocompabible or biodegradable polymer that comprises lactide, glycolide, caprolactone, or a combination thereof.

In specific aspects, the biodegradable polymer can comprise one or more lactide residues. To that end, the polymer can comprise any lactide residue, including all racemic and stereospecific forms of lactide, including, but not limited to, L-lactide, D-lactide, and D,L-lactide, or a mixture thereof Useful polymers comprising lactide include, but are not limited to poly(L-lactide), poly(D-lactide), and poly(DL-lactide); and poly(lactide-co-glycolide), including poly(L-lactide-co-glycolide), poly(D-lactide-co-glycolide), and poly(DL-lactide-co-glycolide); or copolymers, terpolymers, combinations, or blends thereof. Lactide/glycolide polymers can be conveniently made by melt polymerization through ring opening of lactide and glycolide monomers. Additionally, racemic DL-lactide, L-lactide, and D-lactide polymers are commercially available. The L-polymers are more crystalline and resorb slower than DL- polymers. In addition to copolymers comprising glycolide and DL-lactide or L-lactide, copolymers of L-lactide and DL-lactide are commercially available. Homopolymers of lactide or glycolide are also commercially available.

When the biodegradable polymer is poly(lactide-co-glycolide), poly(lactide), or poly(glycolide), the amount of lactide and glycolide in the polymer can vary. In a further aspect, the biodegradable polymer contains 0 to 100 mole %, 40 to 100 mole %, 50 to 100 mole %, 60 to 100 mole %, 70 to 100 mole %, or 80 to 100 mole % lactide and from 0 to 100 mole %, 0 to 60 mole %, 10 to 40 mole %, 20 to 40 mole %, or 30 to 40 mole % glycolide, wherein the amount of lactide and glycolide is 100 mole %. In a further aspect, the biodegradable polymer can be poly(lactide), 95:5 poly(lactide-co-glycolide) 85:15 poly(lactide-co-glycolide), 75:25 poly(lactide-co-glycolide), 65:35 poly(lactide-co-glycolide), or 50:50 poly(lactide-co-glycolide), where the ratios are mole ratios.

In a further aspect, the polymer can be a poly(caprolactone) or a poly(lactide-co-caprolactone). In one aspect, the polymer can be a poly(lactide-caprolactone), which, in various aspects, can be 95:5 poly(lactide-co-caprolactone), 85:15 poly(lactide-co-caprolactone), 75:25 poly(lactide-co- caprolactone), 65:35 poly(lactide-co- caprolactone), 50:50 poly(lactide-co- caprolactone), 40:60 poly(lactide-co-caprolactone), 25:75 poly(lactide-co-caprolactone), 10:90 poly(lactide-co-caprolactone), or 5:95 poly(lactide-co-caprolactone), where the ratios are mole ratios.

It is understood that any combination of the aforementioned biodegradable polymers can be used, including, but not limited to, copolymers thereof, mixtures thereof, or blends thereof. Likewise, it is understood that when a residue of a biodegradable polymer is disclosed, any suitable polymer, copolymer, mixture, or blend, that comprises the disclosed residue, is also considered disclosed. When multiple residues are individually disclosed (*i.e.,* not in combination with another), it is understood that any combination of the individual residues can be used.

In general, any microparticle can be produced by the methods disclosed herein. The micropaticles can have a wide variety of shapes and sizes. In one aspect, the disclosed microparticles can have an average or mean particle size of from about 20 microns to about 125 microns. In one embodiment the range of mean particle size is from about 40 microns to about 90 microns. In another embodiment the range of mean particle sizes is from about 50 microns to about 80 microns. Particle size distributions are measured by laser diffraction techniques known to those of skill in the art.

In a further aspect, as discussed above, the double emulsion process described provides a microparticle comprising the bioactive agent encapsulated, microencapsulated, or otherwise contained within the microparticle. As is known in the art, the microparticle can modulate the release of the bioactive agent, depending on the amount of bioactive agent present in the first aqueous phase. For example, the microparticle can comprise 1%, 5%, 10%, 20%, 30%, 40%, 50%, 60%, 70%, 80%, 90%, 95% by weight bioactive agent, relative to the weight of the microparticle, including any range between the disclosed percentages. It will be apparent that the presently disclosed methods provide, in one aspect, better loading and release profile of a microparticle, relative to a microparticle prepared by a discontinous process. In a specific aspect, the microparticle can comprise at least about 3% bioactive agent by weight, or for example, from about 3% to about 8%, or from about 4% to about 6% bioactive agent.

As will be apparent, depending upon double emulsion processing conditions, the polymer used as a starting material may or may not be the same polymer present in the final microparticle. For example, the polymer used during processing may undergo polymerization or depolymerization reactions, which ultimately can produce a different polymer that was used prior to processing. Thus, the term "polymer" as used herein covers the polymers used as starting materials as well as the final polymer present in the device produced by the methods described herein. Methods for making microparticles can be used in combination with the drying methods and dyring parameters described above.

A wide variety of bioactive agents can be used with the methods described herein. In one aspect, the bioactive agent can be a releasable bioactive agent, *i.e.,* a bioactive agent that can be released from the microparticle into adjacent tissues or fluids of a subject. In certain aspects, the bioactive agent can be in or on the microparticle.

Various forms of the bioactive agent can be used, which are capable of being released from the microparticle into adjacent tissues or fluids. To that end, a liquid or solid bioactive agent can be incorporated into the microparticles described herein. The bioactive agents are at least very slightly water soluble, and preferably moderately water soluble. The bioactive agents can include salts of the active ingredient. As such, the bioactive agents can be acidic, basic, or amphoteric salts. They can be nonionic molecules, polar molecules, or molecular complexes capable of hydrogen bonding. The bioactive agent can be included in the compositions in the form of, for example, an uncharged molecule, a molecular complex, a salt, an ether, an ester, an amide, polymer drug conjugate, or other form to provide the effective biological or physiological activity.

Examples of bioactive agents that incorporated into systems herein include, but are not limited to, peptides, proteins such as hormones, enzymes, antibodies, antibody fragments and the like, nucleic acids such as aptamers, iRNA, DNA, RNA, antisense nucleic acid or the like, antisense nucleic acid analogs or the like, low-molecular weight compounds, or high-molecular-weight compounds. Bioactive agents contemplated for use in the disclosed microparticles include anabolic agents, antacids, anti-asthmatic agents, anti-cholesterolemic and anti-lipid agents, anti-coagulants, anti-convulsants, anti-diarrheals, anti-emetics, anti-infective agents including antibacterial and antimicrobial agents, anti-inflammatory agents, anti-manic agents, antimetabolite agents, anti-nauseants, anti-neoplastic agents, anti-obesity agents, anti-pyretic and analgesic agents, anti-spasmodic agents, anti-thrombotic agents, anti-tussive agents, anti-uricemic agents, anti-vascular growth agents, anti-vascular endothelial growth agents, anti-anginal agents, antihistamines, appetite suppressants, biologicals, cerebral dilators, coronary dilators, bronchiodilators, cytotoxic agents, decongestants, diuretics, diagnostic agents, erythropoietic agents, expectorants, gastrointestinal sedatives, hyperglycemic agents, hypnotics, hypoglycemic agents, immunomodulating agents, ion exchange resins, laxatives, mineral supplements, mucolytic agents, neuromuscular drugs, peripheral vasodilators, psychotropics, sedatives, stimulants, thyroid and anti-thyroid agents, tissue growth agents, vascular growth agents, vascular endothelial growth agents, uterine relaxants, vitamins, or antigenic materials.

Other bioactive agents include androgen inhibitors, polysaccharides, growth factors, hormones, anti-angiogenesis factors, dextromethorphan, dextromethorphan hydrobromide, noscapine, carbetapentane citrate, chlophedianol hydrochloride, chlorpheniramine maleate, phenindamine tartrate, pyrilamine maleate, doxylamine succinate, phenyltoloxamine citrate, phenylephrine hydrochloride, phenylpropanolamine hydrochloride, pseudoephedrine hydrochloride, ephedrine, codeine phosphate, codeine sulfate morphine, mineral supplements, cholestryramine, N-acetylprocainamide, acetaminophen, aspirin, ibuprofen, phenyl propanolamine hydrochloride, caffeine, guaifenesin, aluminum hydroxide, magnesium hydroxide, peptides, polypeptides, proteins, amino acids, hormones, interferons, cytokines, and vaccines.

Representative drugs that can be used as bioactive agents in the microparticles include, but are not limited to, peptide drugs, protein drugs, desensitizing materials, antigens, anti-infective agents such as antibiotics, antimicrobial agents, antiviral, antibacterial, antiparasitic, antifungal substances and combination thereof, antiallergenics, androgenic steroids, decongestants, hypnotics, steroidal anti-inflammatory agents, anti-cholinergics, sympathomimetics, sedatives, miotics, psychic energizers, tranquilizers, vaccines, estrogens, progestational agents, humoral agents, prostaglandins, analgesics, antispasmodics, antimalarials, antihistamines, cardioactive agents, nonsteroidal anti-inflammatory agents, antiparkinsonian agents, antihypertensive agents, β-adrenergic blocking agents, nutritional agents, and the benzophenanthridine alkaloids. The agent can further be a substance capable of acting as a stimulant, sedative, hypnotic, analgesic, anticonvulsant, and the like.

The microparticle can comprise a large number of bioactive agents either singly or in combination. Other bioactive agents include but are not limited to analgesics such as acetaminophen, acetylsalicylic acid, and the like; anesthetics such as lidocaine, xylocaine, and the like; anorexics such as dexadrine, phendimetrazine tartrate, and the like; antiarthritics such as methylprednisolone, ibuprofen, and the like; antiasthmatics such as terbutaline sulfate, theophylline, ephedrine, and the like; antibiotics such as sulfisoxazole, penicillin G, ampicillin, cephalosporins, amikacin, gentamicin, tetracyclines, chloramphenicol, erythromycin, clindamycin, isoniazid, rifampin, and the like; antifungals such as amphotericin B, nystatin, ketoconazole, and the like; antivirals such as acyclovir, amantadine, and the like; anticancer agents such as cyclophosphamide, methotrexate, etretinate, and the like; anticoagulants such as heparin, warfarin, and the like; anticonvulsants such as phenytoin sodium, diazepam, and the like; antidepressants such as isocarboxazid, amoxapine, and the like;antihistamines such as diphenhydramine HCl, chlorpheniramine maleate, and the like; hormones such as insulin, progestins, estrogens, corticoids, glucocorticoids, androgens, and the like; tranquilizers such as thorazine, diazepam, chlorpromazine HCl, reserpine, chlordiazepoxide HCl, and the like; antispasmodics such as belladonna alkaloids, dicyclomine hydrochloride, and the like; vitamins and minerals such as essential amino acids, calcium, iron, potassium, zinc, vitamin B₁₂, and the like; cardiovascular agents such as prazosin HCl, nitroglycerin, propranolol HCl, hydralazine HCl, pancrelipase, succinic acid dehydrogenase, and the like; peptides and proteins such as LHRH, somatostatin, calcitonin, growth hormone, glucagon-like peptides, growth releasing factor, angiotensin, FSH, EGF, bone morphogenic protein (BMP), erythopoeitin (EPO), interferon, interleukin, collagen, fibrinogen, insulin, Factor VIII, Factor IX, Enbrel^{®}, Rituxam^{®}, Herceptin^{®}, alpha-glucosidase, Cerazyme/Ceredose^{®}, vasopressin, ACTH, human serum albumin, gamma globulin, structural proteins, blood product proteins, complex proteins, enzymes, antibodies, monoclonal antibodies, antibody fragments, and the like; prostaglandins; nucleic acids; carbohydrates; fats; narcotics such as morphine, codeine, and the like, psychotherapeutics; anti-malarials, L-dopa, diuretics such as furosemide, spironolactone, and the like; antiulcer drugs such as rantidine HCl, cimetidine HCl, and the like.

The bioactive agent can also be an immunomodulator, including, for example, cytokines, interleukins, interferon, colony stimulating factor, tumor necrosis factor, and the like; allergens such as cat dander, birch pollen, house dust mite, grass pollen, and the like; antigens of bacterial organisms such as *Streptococcus pneumoniae, Haemophilus influenzae, Staphylococcus aureus, Streptococcus pyrogenes, Corynebacterium diphteriae, Listeria monocytogenes, Bacillus anthracis, Clostridium tetani, Clostridium botulinum, Clostridium perfringens. Neisseria meningitides, Neisseria gonorrhoeae, Streptococcus mutans. Pseudomonas aeruginosa, Salmonella typhi, Haemophilus parainfluenzae, Bordetella pertussis, Francisella tularensis, Yersinia pestis, Vibrio cholerae, Legionella pneumophila, Mycobacterium tuberculosis, Mycobacterium leprae, Treponema pallidum, Leptspirosis interrogans, Borrelia burgddorferi, Campylobacter jejuni,* and the like; antigens of such viruses as smallpox, influenza A and B, respiratory synctial, parainfluenza, measles, HIV, SARS, varicella-zoster, herpes simplex 1 and 2, cytomeglavirus, Epstein-Barr, rotavirus, rhinovirus, adenovirus, papillomavirus, poliovirus, mumps, rabies, rubella, coxsackieviruses, equine encephalitis, Japanese encephalitis, yellow fever, Rift Valley fever, lymphocytic choriomeningitis, hepatitis B, and the like; antigens of such fungal, protozoan, and parasitic organisms such as *Cryptococcuc neoformans, Histoplasma capsulatum, Candida albicans, Candida tropicalis, Nocardia asteroids, Rickettsia ricketsii, Rickettsia typhi, Mycoplasma pneumoniae, Chlamyda psittaci, Chlamydia trachomatis, Plasmodium falciparum, Trypanasoma brucei, Entamoeba histolytica, Toxoplasma gondii, Trichomonas vaginalis, Schistosoma mansoni,* and the like. These antigens may be in the form of whole killed organisms, peptides, proteins, glycoproteins, carbohydrates, or combinations thereof.

In a further specific aspect, the bioactive agent comprises an antibiotic. The antibiotic can be, for example, one or more of Amikacin, Gentamicin, Kanamycin, Neomycin, Netilmicin, Streptomycin, Tobramycin, Paromomycin, Ansamycins, Geldanamycin, Herbimycin, Carbacephem, Loracarbef, Carbapenems, Ertapenem, Doripenem, Imipenem/Cilastatin, Meropenem, Cephalosporins (First generation), Cefadroxil, Cefazolin, Cefalotin or Cefalothin, Cefalexin, Cephalosporins (Second generation), Cefaclor, Cefamandole, Cefoxitin, Cefprozil, Cefuroxime, Cephalosporins (Third generation), Cefixime, Cefdinir, Cefditoren, Cefoperazone, Cefotaxime, Cefpodoxime, Ceftazidime, Ceftibuten, Ceftizoxime, Ceftriaxone, Cephalosporins (Fourth generation), Cefepime, Cephalosporins (Fifth generation), Ceftobiprole, Glycopeptides, Teicoplanin, Vancomycin, Macrolides, Azithromycin, Clarithromycin, Dirithromycin, Erythromycin, Roxithromycin, Troleandomycin, Telithromycin, Spectinomycin, Monobactams, Aztreonam, Penicillins, Amoxicillin, Ampicillin, Azlocillin, Carbenicillin, Cloxacillin, Dicloxacillin, Flucloxacillin, Mezlocillin, Meticillin, Nafcillin, Oxacillin, Penicillin, Piperacillin, Ticarcillin, Polypeptides, Bacitracin, Colistin, Polymyxin B, Quinolones, Ciprofloxacin, Enoxacin, Gatifloxacin, Levofloxacin, Lomefloxacin, Moxifloxacin, Norfloxacin, Ofloxacin, Trovafloxacin, Sulfonamides, Mafenide, Prontosil (archaic), Sulfacetamide, Sulfamethizole, Sulfanilimide (archaic), Sulfasalazine, Sulfisoxazole, Trimethoprim, Trimethoprim-Sulfamethoxazole (Co-trimoxazole) (TMP-SMX), Tetracyclines, including Demeclocycline, Doxycycline, Minocycline, Oxytetracycline, Tetracycline, and others; Arsphenamine, Chloramphenicol, Clindamycin, Lincomycin, Ethambutol, Fosfomycin, Fusidic acid, Furazolidone, Isoniazid, Linezolid, Metronidazole, Mupirocin, Nitrofurantoin, Platensimycin, Pyrazinamide, Quinupristin/Dalfopristin, Rifampicin (Rifampin in U.S.), Tinidazole, or a combination thereof. In one aspect, the bioactive agent can be a combination of Rifampicin (Rifampin in U.S.) and Minocycline.

In certain aspects, the bioactive agent can be present as a component in a pharmaceutical composition. Pharmaceutical compositions can be conveniently prepared in a desired dosage form, including, for example, a unit dosage form or controlled release dosage form, and prepared by any of the methods well known in the art of pharmacy. In general, pharmaceutical compositions are prepared by uniformly and intimately bringing the bioactive agent into association with a liquid carrier or a finely divided solid carrier, or both. The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen. Other pharmaceutically acceptable carriers or components that can be mixed with the bioactive agent can include, for example, a fatty acid, a sugar, a salt, a water-soluble polymer such as polyethylene glycol, a protein, polysacharride, or carboxmethyl cellulose, a surfactant, a plasticizer, a high- or low-molecular-weight porosigen such as polymer or a salt or sugar, or a hydrophobic low-molecular-weight compound such as cholesterol or a wax.

The microparticle can be administered to any desired subject. The subject can be a vertebrate, such as a mammal, a fish, a bird, a reptile, or an amphibian. The subject of the herein disclosed methods can be, for example, a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered.

### EXAMPLES

The following examples are put forth so as to provide those of ordinary skill in the art with a complete disclosure and description of how the compounds, compositions, and methods described and claimed herein are made and evaluated, and are intended to be purely exemplary and are not intended to limit the scope of what the inventors regard as their invention. Efforts have been made to ensure accuracy with respect to numbers (e.g., amounts, temperature, *etc*.) but some errors and deviations should be accounted for. Unless indicated otherwise, parts are parts by weight, temperature is in degrees Centigrade (°C) or is at ambient temperature, and pressure is at or near atmospheric. There are numerous variations and combinations of reaction conditions, *e.g.,* component concentrations, component mixtures, desired solvents, solvent mixtures, temperatures, pressures and other reaction ranges and conditions that can be used to optimize the product purity and yield obtained from the described process. Only reasonable and routine experimentation will be required to optimize such process conditions. The polymer used for the following examples was a 75:25 poly(D,L-lactide-co-glycolide) having an intrinsic viscosity of 0.42 dL/g.

### Example 1. Discontinuous double-emulsion (high-speed primary emulsion mixing).

A polymer solution (DP1) was prepared by dissolving 15 grams of polymer in 60 grams of methylene chloride (20% total polymer concentration, by weight). A goserelin solution (DP2) was prepared by dissolving 970 mg goserelin acetate (Genzyme Pharmaceuticals; Cambridge, MA) in 6 mL of water. Separately, a continuous phase (CP) solution was prepared by adding 90 grams of ethyl acetate to 1200 grams of an aqueous solution containing 1 wt% poly(vinyl alcohol), PVA (Amresco, Solon, OH). A primary water-in-oil emulsion (the primary emulsion) was prepared by adding the drug solution DP2 into the polymer solution DP1 and then homogenizing these solutions using three 10-second mixing cycles with an IKA Ultra-Turrax UTL-25 probe mixer fitted with a coarse rotor/stator tip running at a rotor speed of 12,000 RPM. This primary emulsion (the Dispersed Phase (DP) solution) was then immediately placed in a 60-mL syringe which was then placed in a Cole-Parmer dual syringe pump.

The DP solution (the primary emulsion) and the CP solution were separately delivered into the inlet of a laboratory in-line mixer head of a Silverson L4R-T homogenizer fitted with a high-shear disintegrating head stator screen. The DP solution was delivered at a rate of 10 g/min. The CP solution was delivered at a rate of 125 g/min. The Silverson was set to a mixer speed of 1,000 RPM. The effluent emulsion from the Silverson mixer was then immediately diluted with additional extraction-phase water (EP) which was delivered into the effluent emulsion stream at a rate of 3500 grams/min. The resulting effluent was directed through a tube into an 18-gallon tank that was equipped with a suitable mixer (Lightnin G3U05R or similar) set to stir the suspension at about 600-900 RPM. Initially, the bottom valve on the 18-gallon tank was left open and first minute of emulsion product was passed through to waste. After the first minute, a 2-L portion of emulsion was caught from the outlet tubing into a 4-L beaker which was then stirred with magnetic stir bar using a magnetic stir plate. After collecting this portion of the emulsion, the outlet tubing was directed back into the 18-gallon tank where the resulting effluent was passed through the open valve to waste.

Approximately half-way through the run, a second 2-L portion of the product was collected in a 4-L beaker in a similar manner to the first sample. Once collected, the effluent was passed through to waste. Near the end of the run, the bottom valve on the 18-gallon tank was closed and the final portion of the run was collected in this tank. In this manner, three portions of the run were collected from the early, middle, and late portion of the run. All samples were then handled similarly. Each sample was stirred for 90 minutes to permit full solvent extraction from the particles. At this point, each sample was passed across a set of 125 micron and 25 micron test sieves (FisherBrand U.S. Standard stainless steel test sieves) in order to collect the 25-125 micron product that was obtained on the 25 micron screen. Each sample was washed with 4-L deionized water. Next, the product was suspended in 200-mL deionized water, frozen, and lyophilized to remove water (approximately 48 hours) in order to obtain the dry powdered microparticle product. After drying, the microparticle product was transferred to scintillation vial which was securely closed and stored desiccated and frozen until further analysis.

### Example 2. Continuous double-emulsion (high-speed primary emulsion mixing).

The polymer solution (DP1), the aqueous goserelin solution (DP2), and the CP solutions were prepared as described in Example 1. The same IKA probe used in Example 1 (the IKA Ultra-Turrax UTL-25 probe mixer fitted with a coarse rotor/stator tip) was inserted into an IKA in-line mixer head adapter in order to operate in a continuous mixing mode. Tubing was configured to allow both the DP1 polymer solution and DP2 aqueous goserelin solution to be individually introduced into the inlet of the in-line mixer head adapter. By delivering both the DP1 and DP2 solutions into the in-line mixer head at the same time, the primary emulsion was able to be generated in a continuous manner. The DP1 polymer solution was pumped at a rate of 10 g/min while the DP2 goserelin solution was delivered at a rate of 1 mL/min; the IKA mixer was set to a mixer speed of 12,000 RPM. The resulting primary emulsion (the DP solution) was therefore produced at a rate of about 11 g/min in a continuous manner. The primary emulsion (the DP solution) was then delivered in a continuous manner directly to the inlet of a laboratory in-line mixer head of a Silverson L4R-T homogenizer along with the CP solution as described in Example 1. The CP solution was delivered at a rate of 125 g/min. The run and the collection of the three fractions from the early, middle, and late portion of the run were performed as described in Example 1. Samples were collected, washed, and dried as described in Example 1.

Drug (goserelin) content was performed on selected samples by HPLC. In vitro release was performed by incubating samples at 37°C in phosphate-buffered saline solutions and analyzing portions of the release medium by HPLC at the indicated time intervals. In each case, portions of the samples were analyzed from the middle and the late portion of each run wherein processing conditions had reached steady-state.

Scanning electron microscopy (SEM) was performed on select samples. Microparticle cross sections were performed using a cryo-microtome technique. Confocal raman was utilized to look at distribution of components inside and throughout the bulk of the individual microparticles.

The product made from the discontinuous primary emulsion process exhibited relatively large initial burst, likely a result from the variability in the morphology of the resulting microparticle product. The population of particles having poorly-distributed drug entrapped within the particles produced relatively large burst release of goserelin. In contrast, the improved and relatively consistent cross-sectional morphology of particles formed by the continuous double-emulsion process resulted in a reduction in burst. This was likely a result of the controlled and improved distribution of drug throughout the polymer matrix along with excellent reproducibility and consistency in drug distribution and particle attributes throughout the run. The performance data for the microparticles of Examples 1 and 2 are summarized in Table 1.

**Table 1. Characterization of formulations from Examples 1 and 2.**

| Example | Processing conditions | Portion of run | Theoretical drug loading | Measured drug loading | In vitro release, cumulative percent | | |
|---|---|---|---|---|---|---|---|
| | | | | | 2 hours | 24 hours | 48 hours |
| 1 | Discontinuous primary emulsion; 12,000 RPM primary emulsion mixing | Middle | 6% | 5.6% | 12.3 | 20.9 | 29.7 |
| | | Late | 6% | 5.9% | 14.6 | 23.3 | 30.4 |
| 2 | Continuous primary emulsion; 12,000 RPM primary emulsion mixing | Middle | 6% | 5.3% | 0 | 12.9 | 15.2 |
| | | Late | 6% | 5.25 | 0 | 14.8 | 16.2 |

### Example 3. Discontinuous double-emulsion process (high ratio of inner-phase water used in the preparation of the primary emulsion).

A formulation was prepared in a manner similar to the discontinuous double-emulsion process of Example 1 except that a larger ratio of water was used during preparation of the primary emulsion. In this case, a polymer solution (DP1) was prepared by dissolving 20 grams of polymer in 80 grams of methylene chloride (20% total polymer concentration, by weight). A goserelin solution (DP2) was prepared by dissolving 1.29 g in 20mL of water. Separately, a continuous phase (CP) solution was prepared by adding 90 grams of ethyl acetate to 1200 grams of an aqueous solution containing lwt% poly(vinyl alcohol) PVA (Amresco, Solon, OH). The two solutions were homogenized together (as described in Example 1) using an IKA probe mixer at 12,000 rpm to form the dispersed phase (DP) solution (the primary emulsion). This emulsion was then placed inside a 100-mL syringe which, in turn, was placed in a Cole-Parmer dual syringe pump. The DP solution and the CP solution were then delivered into and homogenized using a Silverson L4R-T homogenizer as described in Example 1. The remaining steps were also performed as described in Example 1 in order to obtain samples of the microparticle product from the early, middle, and late portions of the run.

### Example 4. Continuous double-emulsion process (high ratio of inner-phase water used in the preparation of the primary emulsion).

A formulation was prepared in a manner similar to the continuous double-emulsion process of Example 2 except that a larger ratio of water was used during preparation of the primary emulsion. In this case, the polymer, goserelin and CP solutions were prepared as described in Example 3. Other processing steps were then carried out as described in Example 2 for preparation of the microparticles using a continuous double-emulsion process. Portions of the product were collected from the early, middle, and late portions of the run run as described in Example 2.

Example 4 samples had very high loadings of the water-soluble drug (encapsulation efficiencies) despite being prepared with relatively large quantities of inner-phase water. This was not observed when samples were prepared from a discontinuous primary emulsion (Example 3). The continuous process of Example 4 produced a product that only slowly released drug over the first 48-hours of the in vitro release system. This demonstrates the relatively improved and consistent drug distribution and encapsulation provided by the continuous double-emulsion process (as compared to the discontinuous process used in Example 3). The performance data for the microparticles of Examples 3 and 4 are summarized in Table 2. Drug content, in vitro release, and SEMs were measured using the same protocol as in Examples 1 and 2 above.

**Table 2. Characterization of formulations from Examples 3 and 4.**

| Example | Processing conditions | Portion of run | Theoretical drug loading | Measured drug loading | In vitro release, cumulative percent | | |
|---|---|---|---|---|---|---|---|
| | | | | | 2 hours | 24 hours | 48 hours |
| 3 | Discontinuous primary emulsion; 12,000 RPM primary emulsion mixing | Middle | 6% | 3.4 | 25.0 | 26.3 | 47.4 |
| | | Late | 6% | 3.2 | 25.9 | 26.4 | 47.8 |
| 4 | Continuous primary emulsion; 12,000 RPM primary emulsion mixing | Middle | 6% | 6.8 | 14.1 | 14.3 | 26.2 |
| | | Late | 6% | 6.5 | 13.6 | 13.9 | 25.5 |

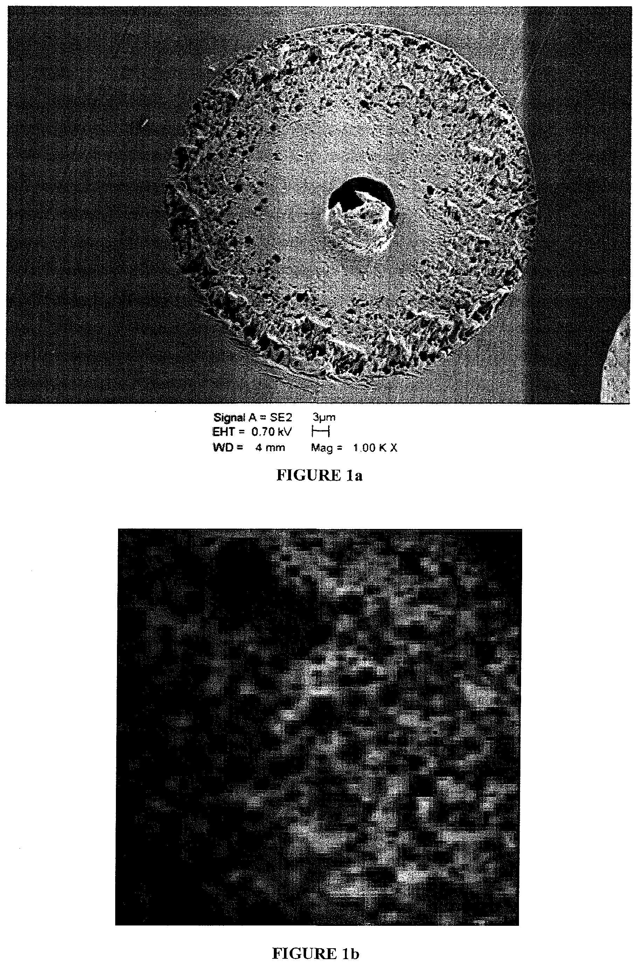

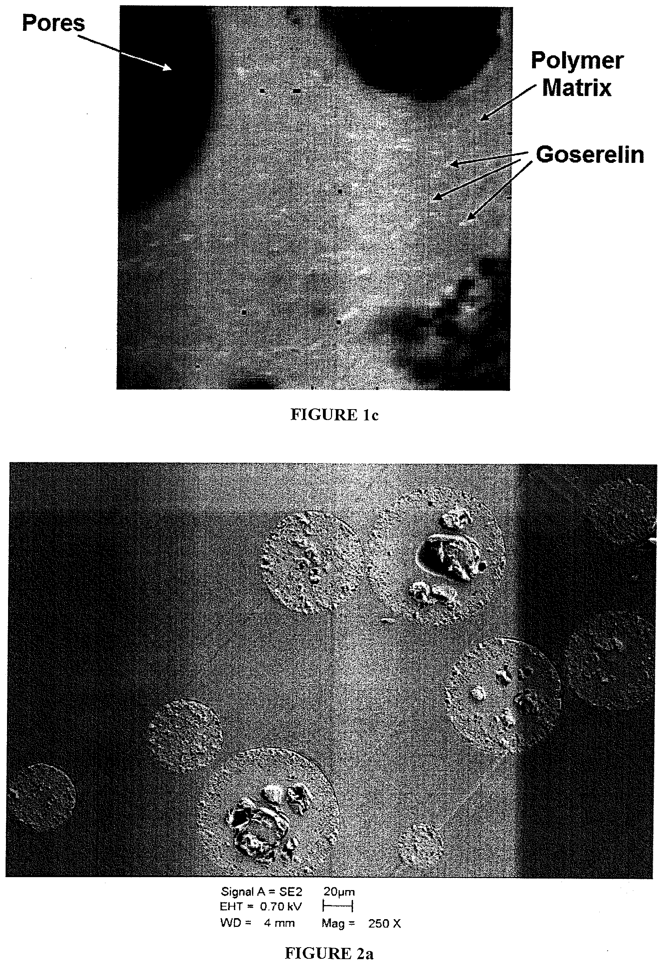

With reference to Figure 1, it can be seen that microparticles prepared herein by the discontinuous process described in Example 1 were observed by SEM cross-sections to have varying amounts and distributions of pores or holes. Figure 1a shows a representative SEM cross section of pores formed near the surface of an individual microparticle. Confocal raman analysis, shown in Figure 1b, provides supporting evidence that there was drug concentrated around the periphery of these pores as well as being distributed in and throughout the polymer matrix as shown in Figure 1c. In Figure 1, the grey area represents the polymer matrix; black represents the air-filled voids or pores; light grey (white to light grey) represents drug (goserelin).

These observations may provide evidence that these pores represent the voids left behind from larger droplets of the aqueous phase of the primary emulsion (the drug-containing aqueous solution) that were formed during particle formation. Otherwise, drug in all cases was found to be evenly distributed throughout the remainder of the polymer-rich (void-free) matrix as demonstrated in Figure 1 (c). Thus, it is likely that the polymer-rich regions inside particles were formed from a very finely-dispersed drug-containing primary emulsion. In contrast, the voids or pores were formed from droplets of the primary emulsion that had coalesced to form larger aqueous droplets as quality of the primary emulsion slowly degraded.

With reference to Figures 2a-c, it can be seen that cross-sections of microparticles removed from various portions of the microparticle process of Example 1 exhibit varied attributes. Some are nearly solid (non-porous) while a majority of particles appear porous across their diameters. Further, the porous particles vary in terms of their pore sizes: some particles exhibit fine porosity while other particles exhibit fine-to-course porosity. Generally, however, microparticles prepared with a discontinuous primary emulsion process are quite variable and inconsistent in their internal morphology.

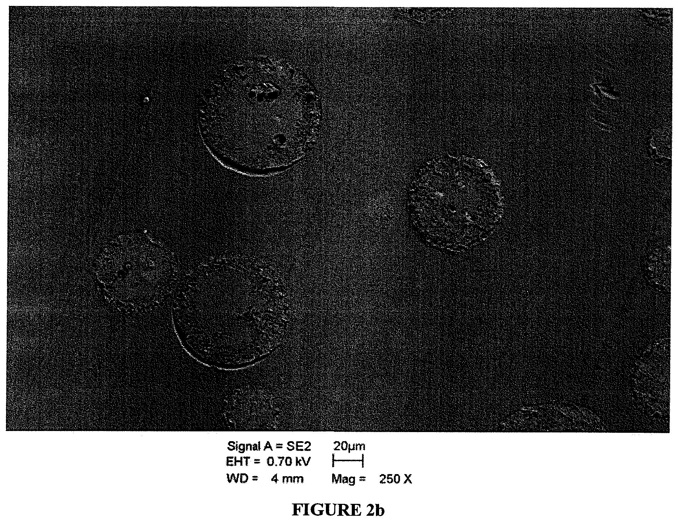

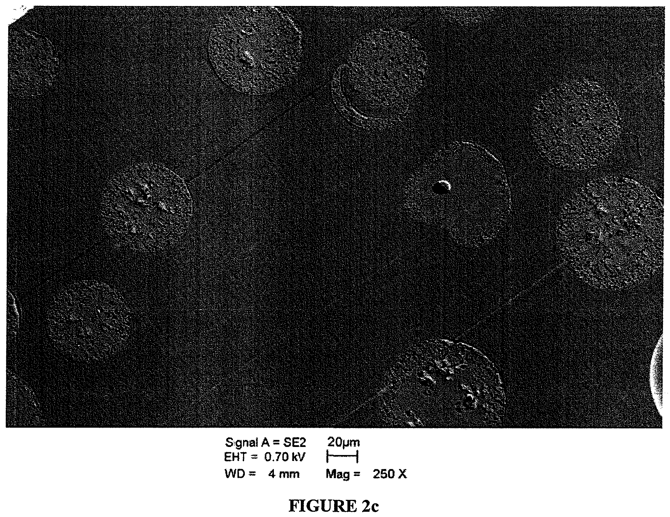

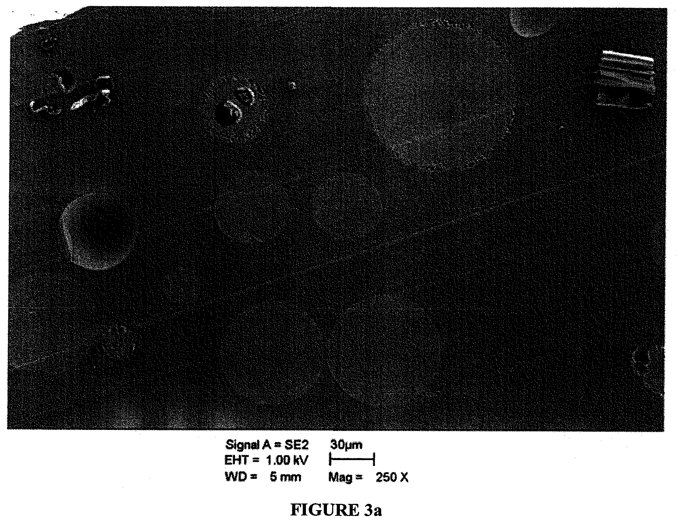

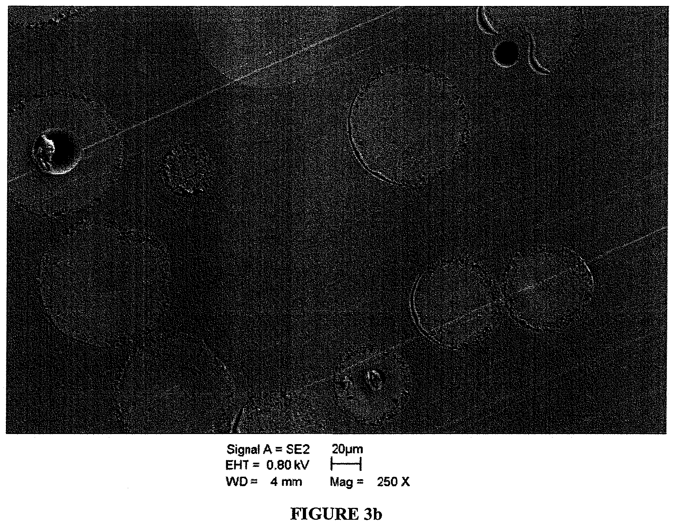

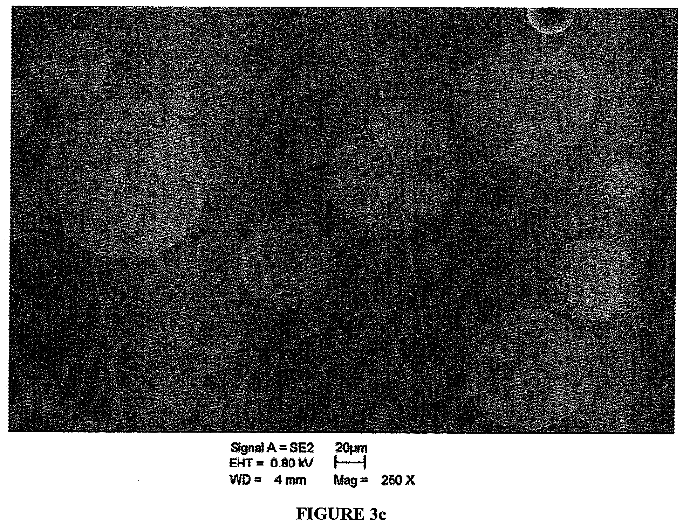

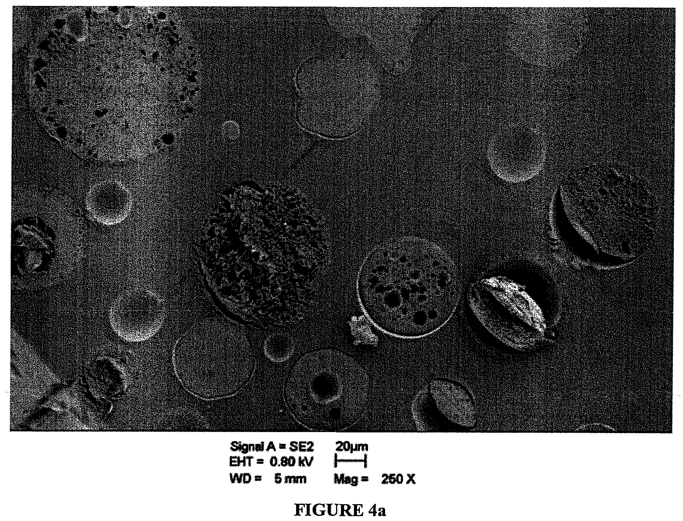

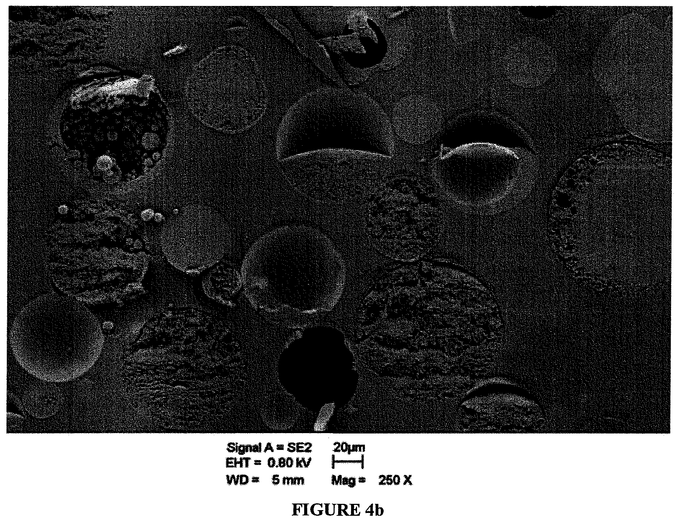

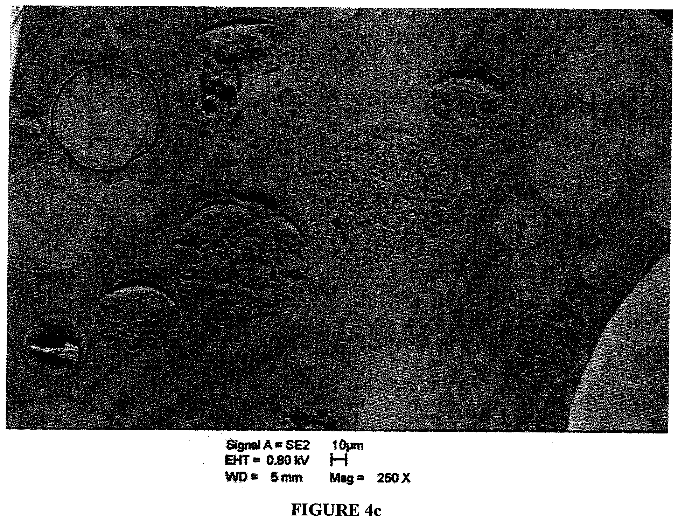

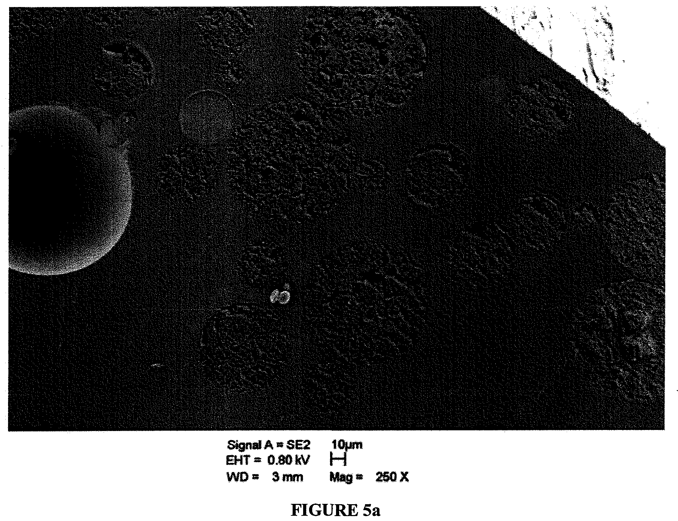

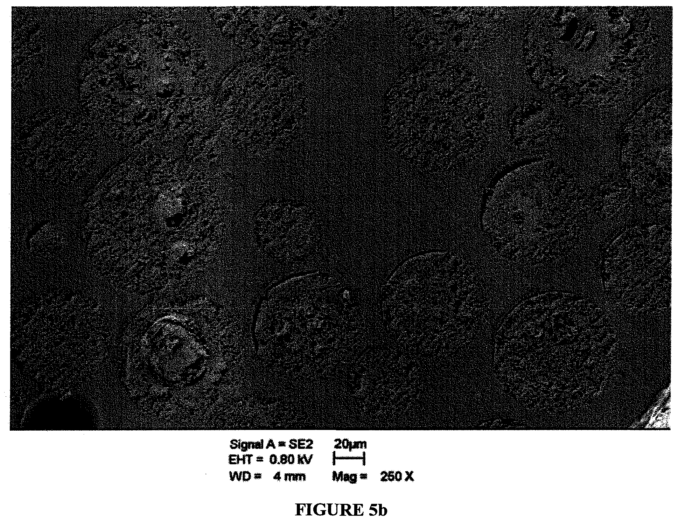

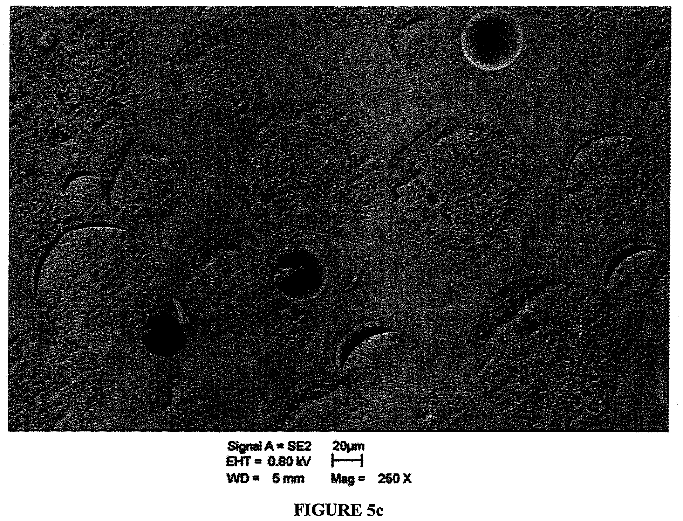

In contrast to the discontinuous double-emulsion process of Example 1, the continuous preparation of the primary emulsion used to prepare the sample of Example 2 produced a relatively well-defined primary emulsion as was evidenced by the lack of voids or pores on the interior of these particles. With reference to Figures 3a-c, SEM images of particles from Example 2 (a continuous double-emulsion using 12,000 RPM for preparation of the primary emulsion) are provided. As mentioned in reference to Figure 1, confocal raman (data not shown) images show drug particles that are homogeneous in size and that are well distributed throughout the bulk polymer maxtrix of the individual particle. Furthermore, the interior morphology of the particles remains virtually identical from particle-to-particle across the entire run. This inter-particle consistency demonstrates the advantage of control and reproducibility in product attributes by using a continuous double-emulsion process. With reference to Figures 4a-c and Figures 5a-c, and similar to those of Figures 2 and 3 (from Examples 1 and 2), it can be seen that the particles formed using the discontinuous process (Example 3) (Figures 4a-c) are inconsistent and variable in the cross-sectional morphology. In contrast, the particles formed from the continuous double-emulsion process (Example 4) (Figures 5a-c) are more consistent in their cross-sectional appearance and morphology.

Various modifications and variations can be made to the compounds, composites, kits, articles, devices, compositions, and methods described herein. Other aspects of the the compounds, composites, kits, articles, devices, compositions, and methods described herein will be apparent from consideration of the specification and practice of the the compounds, composites, kits, articles, devices, compositions, and methods disclosed herein. It is intended that the specification and examples be considered as exemplary.

## Claims

1. A method for forming microparticles comprising:
(a) providing a first phase comprising a bioactive agent dissolved or dispersed in a first aqueous phase;
(b) providing a second phase comprising a polymer dissolved or dispersed in an organic phase;
(c) continuously mixing the first and second phases to form a water-in-oil emulsion;
(d) continuously mixing the emulsion of step (c) with a second aqueous phase to form a water-in-oil-in-water double emulsion; and
(e) removing the organic to form the microparticles.

2. The method of claim 1, wherein the bioactive agent is water-soluble or water-dispersible.

3. The method of claim 1 or 2, wherein steps (c) and (d) are each independently carried out using a static mixer, a rotor/stator homogenizer, or a combination thereof.

4. The method of any preceding claim, wherein removing the organic comprises extracting the organic with an extraction liquid.

5. The method of any preceding claim, wherein the polymer is poly(lactide), poly(glycolide), poly(caprolactone), or a copolymer thereof.

6. The method of any preceding claim, wherein the polymer is poly(lactide-co-glycolide).

7. The method of any preceding claim, wherein the polymer is a 75:25 poly(D,L-lactide-co-glycolide).

8. The method of claim 7, wherein the polymer has an intrinsic viscosity of 0.42 dL/g.

9. A microparticle made by the method of any preceding claim.

## Patentansprüche

1. Verfahren zum Herstellen von Mikropartikeln, umfassend:
(a) Bereitstellen einer ersten Phase, die ein bioaktives Mittel umfasst, das in einer ersten wässrigen Phase gelöst oder dispergiert ist;
(b) Bereitstellen einer zweiten Phase, die ein Polymer umfasst, das in einer organischen Phase gelöst oder dispergiert ist;
(c) kontinuierliches Mischen der ersten mit der zweiten Phase, um eine Wasser-in-Öl-Emulsion zu bilden;
(d) kontinuierliches Mischen der Emulsion aus Schritt (c) mit einer zweiten wässrigen Phase, um eine Wasser-in-Öl-in-Wasser-Doppelemulsion zu bilden; und
(e) Entfernen der organischen Stoffe, um die Mikropartikel zu bilden.

2. Verfahren gemäß Anspruch 1, wobei das bioaktive Mittel wasserlöslich oder wasserdispergierbar ist.

3. Verfahren gemäß Anspruch 1 oder 2, wobei die Schritte (c) und (d) jeweils unabhängig voneinander unter Verwendung eines statischen Mischers, eines Rotor/Stator-Homogenisators oder einer Kombination davon durchgeführt werden.

4. Verfahren gemäß einem der vorstehenden Ansprüche, wobei das Entfernen der organischen Stoffe das Extrahieren der organischen Stoffe mit einer Extraktionsflüssigkeit umfasst.

5. Verfahren gemäß einem der vorstehenden Ansprüche, wobei das Polymer Poly(lactid), Poly(glycolid), Poly(caprolacton) oder ein Copolymer davon ist.

6. Verfahren gemäß einem der vorstehenden Ansprüche, wobei das Polymer Poly(lactid-co-glycolid) ist.

7. Verfahren gemäß einem der vorstehenden Ansprüche, wobei das Polymer ein 75:25 Poly(D,L-lactid-co-glycolid) ist.

8. Verfahren gemäß Anspruch 7, wobei das Polymer eine intrinsische Viskosität von 0,42 dl/g aufweist.

9. Mikropartikel, hergestellt durch das Verfahren gemäß einem der vorstehenden Ansprüche.

## Revendications

1. Procédé de formation de microparticules comprenant:
(a) la fourniture d'une première phase comprenant un agent bioactif dissous ou dispersé dans une première phase aqueuse;
(b) la fourniture d'une deuxième phase comprenant un polymère dissous ou dispersé dans une phase organique ;
(c) le mélange en continu de la première avec la deuxième phase pour former une émulsion eau dans l'huile ;
(d) le mélange en continu de l'émulsion de l'étape (c) avec une deuxième phase aqueuse pour former une double émulsion eau dans l'huile dans l'eau ; et
(e) le retrait des matières organiques pour former les microparticules.

2. Procédé de la revendication 1, dans lequel l'agent bioactif est hydrosoluble ou dispersible dans l'eau.

3. Procédé de la revendication 1 ou 2, dans lequel les étapes (c) et (d) sont, chacune indépendamment, conduites au moyen d'un mélangeur statique, d'un homogénéisateur à rotor/stator, ou d'une combinaison de ceux-ci.

4. Procédé de l'une quelconque des revendications précédentes, dans lequel le retrait de la phase organique comprend l'extraction des matières organiques avec un liquide d'extraction.

5. Procédé de l'une quelconque des revendications précédentes, dans lequel le polymère est un poly(lactide), un poly(glycolide), une poly(caprolactone), ou un copolymère de ceux-ci.

6. Procédé de l'une quelconque des revendications précédentes, dans lequel le polymère est un poly(lactide-co-glycolide).

7. Procédé de l'une quelconque des revendications précédentes, dans lequel le polymère est un poly(D,L-lactide-co-glycolide) 75:25.

8. Procédé de la revendication 7, dans lequel le polymère a une viscosité intrinsèque de 0,42 dl/g.

9. Microparticule fabriquée par le procédé de l'une quelconque des revendications précédentes.
